# EUROPEAN PATENT SPECIFICATION

(11) **EP 1 353 181 B1**
(45) Date of publication and mention of the grant of the patent: **04.07.2012**
(21) Application number: 02760610.2
(22) Date of filing: 12.08.2002
(51) Int. Cl.: G01N 33/558

(54) **BIOSENSOR AND METHOD FOR ANALYZING BLOOD COMPONENTS USING IT**
BIOSENSOR UND DESSEN VERFAHREN ZUR ANALYSE VON BLUTBESTANDTEILEN
BIODETECTEUR ET PROCEDE SERVANT A ANALYSER DES CONSTITUANTS SANGUINS AU MOYEN DE CE DERNIER

(30) Priority: 10.08.2001 JP 2001243855
(43) Date of publication of application: 15.10.2003
(73) Proprietor: Panasonic Corporation, Kadoma-shi Osaka 571-8501 (JP)
(72) Inventor: TAKAHASHI, Mie, Nihama-shi, Ehime 792-0026 (JP); NADAOKA, Masataka, Iyo-shi, Ehime 799-3113 (JP); TANAKA, Hirotaka, Matsuyama-shi, Ehime 791-1102 (JP); KITAWAKI, Fumihisa, Kadoma-shi, Osaka 571-0064 (JP)
(74) Representative: Balsters, Robert
(86) International application number: PCT/JP2002/008208
(87) International publication number: WO 2003/014741

(56) References cited:
- EP-A- 0 903 584
- EP-A- 1 202 059
- WO-A-01/31340
- DE-A1- 10 018 959
- JP-A- 5 005 743
- JP-A- 7 055 809
- JP-A- 7 103 973
- JP-A- 2001 013 141
- JP-U- 5 077 763
- US-A- 5 628 890

## Description

### TECHNICAL FIELD

The present invention relates to a biosensor and, more particularly, to a dry-type biosensor for analyzing an analyte in a sample solution, and a method for analyzing a constituent of blood by employing the biosensor.

### BACKGROUND ART

As a means for diagnosing health conditions of persons, a biochemical examination of bodily fluids, especially blood, is widely conducted. A measuring method by a chromatography sensor, which utilizes an antigen-antibody reaction is generally used as a method for analyzing the bodily fluids. However, it is difficult to determine a kind or measure the concentration of blood constituent such as a metabolic product, a protein, a fat, an electrolyte, an enzyme, an antigen, and an antibody, by employing whole blood as it is. Thus, conventionally a general blood constituent analysis method requires several operation processes, such as centrifuging blood which is previously collected, and analyzing the blood constituent employing obtained blood plasma or blood serum by an analytical instrument or a biosensor. However, when this method is employed, the measurement requires a special apparatus, and the preprocessing as well as the inspection take a lot of time. Thus, the centrifugation method which requires a centrifugal machine is not adequate when a small number of specimens are to be processed quickly or when inspections in the field is performed. Further, the obtained blood serum or blood plasma is smaller in amount as compared with the amount of blood.

In recent years, a device which enables a quick, simple, and accurate measurement is desired under a concept of POC (Point of Care) in the medical diagnosis scene. However, in the conventional method as described above, in the case where a sample solution is to be applied to a sensor part, for example, when the sample solution is blood, a series of operations as described below is required. That is, blood is collected by the use of an injector, blood cells as material components and blood plasma are separated by the use of a centrifugal machine or the like in general, and the separated blood is applied to the sensor part with the use of an instrument such as a dispenser and a dropper. In this method, special skills in medical technology or the like are required to collect blood employing an injector, and further special apparatus and skills are required for the operation of centrifugal separation, and therefore this method cannot be employed in general households or when individuals without such techniques perform measurements for themselves. Further, since the instrument such as a dispenser is required to quantitatively measure the sample solution, the operation becomes complicated.

Then, a method for separating blood plasma from whole blood by filtration has been considered. For example, there are a blood cell separation method employing glass fiber filter paper of a certain density as disclosed in Japanese Published Patent Application No. Sho. 57-53661 and No. Hei.8-54387, and a blood adjustment method in which a water solution of inorganic salt or amino acid of a certain concentration is applied to whole blood and then blood cell components are filtered, as disclosed in Japanese Published Patent Application No. Hei.9-196908.

In the method as disclosed in Japanese Published Patent Application No. Sho.57-53661 and No. Hei.8-54387, in order to more completely separate the blood cell components, glass fiber filter paper having an average diameter of 0.2~5µm and density of 0.1∼0.5g/cm³ is employed to make blood ooze therefrom, thereby obtaining separated blood plasma or blood serum. However, when this method is employed, while the efficiency of blood cell separation is surely enhanced, considerable time is required for almost completely separating the blood cells, and a large amount of blood is required to obtain a specimen in amount required for the inspection.

Further, in the method as disclosed in Japanese Published Patent Application No. Hei.9-196908, in order to avoid clogging of the filtration material due to the blood cells, and to obtain a larger amount of blood plasma or blood serum component employing a smaller amount of blood, the water solution of amino acid or inorganic salt is mixed with whole blood, and thereafter the blood cell components are filtered. When this method is employed, operations of previously adding to obtained blood the water solution to be applied, and thereafter filtering the blood cell components are required, whereby the operation becomes complicated and the measurement takes time. Thus, inspections in emergency situations cannot be dealt with.

Furthermore, in a method as disclosed in WO 01/92886A1, a cell contraction agent is employed to cause the blood cell components to constrict, and the constricted blood cell components are developed on a chromatography carrier. When this method is employed, the constricted blood cell components can be developed with blood plasma components, thereby requiring no preprocessing on the specimen. However, it is difficult to uniformly constrict the blood cell components by the cell contraction agent, resulting in a poor measurement reproducibility.

The present invention is made to solve the above-mentioned problems and has for its object to provide a simple, quick, and high-performance biosensor which can confirm the result of blood constituent analysis only by applying a slight amount of blood thereto, without requiring special apparatus, and a method for analyzing a blood constituent by employing the biosensor.

JP-A-05 005 743 discloses an immunity measuring apparatus provided with a filter, a member containing a labelling reagent and a porous carrier with one or a plurality of reaction areas. The porous carrier is held between the filter and the member at the upstream end. A reaction area is located downstream, separated from the filter and the member. The member contains a labelled reagent and a scavenger reagent is fixed to the reaction area.

EP-A-0 903 584 discloses immunochromatography-assisted device comprising a porous carrier having an additive-impregnated part between a sample introducing part and a determining part, the additive-impregnating part carrying at least one selected from the group consisting of a surfactant, a water-soluble ammonium salt and a pH buffer such that is dissolves in a sample introduced into the carrier in order to move with the movement of the sample through the carrier. The porous carrier also has a labelled part segmented into plural zones.

### DISCLOSURE OF THE INVENTION

According to Claim 1 of the present invention, there is provided a biosensor made of a dried porous material. The biosensor comprises: a sample introductory part for introducing a sample solution; and a developing layer for developing the sample solution, and in this biosensor the developing layer includes: a marker reagent holding part where a marker reagent is held in a dry state so that it can be eluted by the development of the sample solution; and a reagent immobilization part where a reagent which can bind to an analyte and is involved in a reaction is immobilized so that it is not eluted, and when the sample solution is introduced to the sample introductory part, the sample solution permeates the developing layer to reach the marker reagent holding part, and moves to the reagent immobilization part while eluting the marker reagent, whereby the reaction among the analyte, the marker reagent, and the immobilized reagent occurs, and an amount of marker reagent bound in the reagent immobilization part is measured, thereby qualitatively or quantitatively measuring the analyte included in the sample solution. In this biosensor a retiform structure is arranged in the sample introductory part.

According to the present invention, the applied sample solution is developed on the biosensor while being agitated by a turbulent flow caused by the retiform structure, whereby the permeability of a reactive layer carrier is enhanced, and a more uniform permeation is realized, resulting in a biosensor which enables a simple, quick, more sensitive, and higher-performance measurement.

The retiform structure as mentioned above is formed by performing a molding process on a fiber or resin to reticulate the same, and it makes no difference whether the retiform structure itself has capillary activity or absorbability or not. The reticulum at this time may have any shape as long as it is polygonal, and the size thereof does not matter. It is preferable that meshes are arranged in regular sizes. Further, this retiform structure is preferably a single layer.

Further, the turbulent flow is a flow in which a fluid irregularly moves in disorder and a stream line shows a fine and irregular fluctuation.

According to the present invention, the biosensor is made of a dried porous material. The sample introductory part of said biosensor is provided with a space forming part for forming a space on the developing layer so as to produce cavity into which the sample solution is sucked and held therein.

According to the present invention, a specific amount of sample solution is sucked in the sample introductory part as the cavity formed by the space forming material, and the applied sample solution is developed on the biosensor while being efficiently agitated by a turbulent flow generated by the reriform tissue. Therefore, the permeability of a reactive layer carrier is enhanced, and a more uniform permeation is realized, resulting in a biosensor which enables a simple, quick, more sensitive, and higher-performance measurement.

According to the present invention a cell contraction agent holding part holding cell contraction agent which contracts cellular components is included in the space forming part.

According to the present invention, there no need to previously remove the cellular components in the sample solution or fragmentizing the same, thereby realizing a biosensor which enables a simpler and quicker measurement.

According to the present invention, the retiform structure comprises a single layer with a mesh having a pore size of 0.1 mm to 2 mm arranged in the sample introductory part.

According to the present invention, clogging by the cellular component is avoided, and the sample solution is efficiently agitated by a turbulent flow generated by the retiform structure. Therefore, a smaller amount of sample solution realizes a uniform permeation on a reactive layer carrier, resulting in a biosensor which enables a simple, quick, sensitive, and high-performance measurement.

According to Claim 2 of the present invention, the marker reagent holding part is disposed in the cavity, and the sample solution is introduced to the sample introductory part, the sample solution is developed in the developing layer while the marker reagent is eluted to reach the reagent immobilization part, thereby occurring a reaction among the analyte, the marker reagent, and the immobilized reagent.

According to the present invention, a specific amount of sample solution is sucked in the sample introductory part as the cavity formed by the space forming material, and the applied sample solution is developed on the biosensor while being efficiently agitated by a turbulent flow generated by the reriform tissue and, thus, more thoroughly reacting with the marker reagent. Therefore, the permeability of a reactive layer carrier is enhanced, and a more uniform permeation is realized, resulting in a biosensor which enables a simple, quick, more sensitive, and higher-performance measurement.

According to Claim 3 of the present invention, in the biosensor as defined in Claims 1, the retiform structure is arranged at the end part of the sample introductory part.

According to the present invention, as soon as the sample is applied, the applied sample solution is developed on the biosensor while being efficiently agitated by a turbulent flow generated by the retiform structure, whereby a more uniform and effective cellular constriction is performed, and the sample solution permeates a reactive layer carrier uniformly, resulting in a biosensor which enables a simple, quick, more sensitive, and higher-performance measurement.

According to Claim 4 of the present invention, in the biosensor as defined in Claim 1, the retiform structure and the cell contraction agent holding part are arranged so that the edges of the end parts thereof are kept aligned.

According to the present invention, the introduced sample solution can immediately react with the cell contraction agent as well as receive the effect of turbulent flow at the introduction, whereby the sample solution can efficiently react with the cell contraction agent. Therefore, a more uniform and effective cellular constriction is performed, and the sample solution permeates a reactive layer carrier uniformly, resulting in a biosensor which enables a simple, quick, more sensitive, and higher-performance measurement.

According to Claim 5 of the present invention, in the biosensor as defined in Claim 1, a space enabling the sample solution to flow therein is arranged between the retiform structure and the cell contraction agent holding part.

According to the present invention, the sample solution is smoothly introduced, and a sufficient constriction reaction which occurs by means of the cell contraction agent and effect of turbulent flow due to the retiform structure can be obtained, whereby a more uniform and effective cellular constriction is performed, and the sample solution permeates a reactive layer carrier uniformly, resulting in a biosensor which enables a simple, quick, more sensitive, and higher-performance measurement.

According to Claim 6 of the present invention, in the biosensor as defined in Claim 1, the retiform structure is arranged so that warp threads thereof are parallel to the direction in which the sample is developed on the developing layer.

According to the present invention, the sample solution which is developed while being efficiently agitated by a turbulent flow is efficiently led in the direction of the development by a capillary phenomenon caused by warp threads of the retiform structure that extend in the direction of the sample development. Therefore, the sample solution permeates a reactive layer carrier uniformly, resulting in a biosensor which enables a simple, quick, more sensitive, and higher-performance measurement.

The warp thread as mentioned above is a part of the retiform structure in which a molded product of a fiber or resin forming the reticulum extends in the direction of the development. It is preferable that the warp thread is regularly arranged so that it is parallel to the direction of the development. Directions of tissues extending in other directions than that described above do not matter.

According to Claim 7 of the present invention, in the biosensor as defined in Claim 1, the reitform tissue is made of synthetic resin.

According to the present invention, the retiform structure itself lacks the water retaining capacity, whereby the sample solution is well drained, and the applied sample solution is quickly developed on the biosensor while being efficiently agitated by a turbulent flow generated by the retiform structure. Therefore, a smaller amount of sample solution realizes a uniform permeation on a reactive layer carrier, resulting in a biosensor which enables a simple, quick, sensitive, and high-performance measurement.

According to Claim 8 of the present invention, in the biosensor as defined in Claim 1, the retiform structure is made of a chemical fiber such as polyester.

According to the present invention, the retiform structure itself lacks the water retaining capacity, whereby the sample solution is well drained, and the applied sample solution is efficiently and quickly developed on the biosensor by a capillary phenomenon, while being efficiently agitated by a turbulent flow generated by the retiform structure. Therefore, a smaller amount of sample solution realizes a uniform permeation on a reactive layer carrier, resulting in a biosensor which enables a simple, quick, sensitive, and high-performance measurement.

The retiform structure as mentioned above is formed by performing a welding process such as a thermocompression bonding and a press work on a chemical fiber, to reticulate the same.

According to Claim 9 of the present invention, in the biosensor as defined in Claim 1, the retiform structure is a fabric obtained by weaving the chemical fiber such as polyester.

According to the present invention, the retiform structure itself lacks the water retaining capacity, whereby the sample solution is well drained, and the applied sample solution is efficiently and quickly developed on the biosensor by a capillary phenomenon, while being efficiently agitated by a turbulent flow generated by the retiform structure. Therefore, a smaller amount of sample solution realizes a uniform permeation on a reactive layer carrier, resulting in a biosensor which enables a simple, quick, sensitive, and high-performance measurement.

The retiform structure as mentioned above is a fabric or a knit formed by the process of weaving or knitting a chemical fiber.

According to Claim 10 of the present invention, in the biosensor as defined in Claim 1, the retiform structure is treated with a surfactant so that it can permeate.

According to the present invention, the retiform structure does not repel the liquid sample, and the applied sample solution is quickly developed on the biosensor while being efficiently agitated by a turbulent flow generated by the retiform structure. Therefore, a smaller amount of sample solution realizes a uniform permeation on a reactive layer carrier, resulting in a biosensor which enables a simple, quick, sensitive, and high-performance measurement.

According to Claim 11 of the present invention, in the biosensor as defined in any of Claim 1, the sample solution to be applied is blood.

According to the present invention, there is no need to previously subject blood to some processing, thereby realizing a biosensor enabling a simple, quick, sensitive, and high-performance measurement, by which a safer and more sanitary blood examination can be conducted.

According to Claim 12 of the present invention, in the biosensor as defined in Claim 1, the sample solution to be applied is a solution including bacteria.

According to the present invention, there is no need to subject the solution including bacteria to some processing, thereby realizing a biosensor which enables a safer, more sanitary, simple, quick, sensitive, and high-performance measurement.

According to Claim 13 of the present invention, in the biosensor as defined in Claim 1, the cell contraction agent is inorganic salt.

According to the present invention, the cellular components in the liquid solution are constricted, so that clogging by the cellular components in the liquid sample such as whole blood or a bacterial solution is avoided, thereby realizing a uniform permeation state. Therefore, a biosensor which is able to perform a more accurate measurement in a short time without inhibiting the reaction is realized. The inorganic salt as mentioned above is an inorganic compound including salt, such as sodium chloride, potassium chloride, and sodium phosphate.

According to Claim 14 of the present invention, in the biosensor as defined in Claim 1, the cell contraction agent is amino acid.

According to the present invention, the cellular components in the liquid solution are constricted, so that clogging by the cellular components in the liquid sample such as whole blood or a bacterial solution is avoided, thereby realizing a uniform permeation state. Therefore, a biosensor which is able to perform a more accurate measurement in a short time without inhibiting the reaction is realized. The amino acid as mentioned above is a compound which has a carboxyl group and an amino group in an identical molecule, such as glycin and glutamic acid, and further includes imino acid such as proline and hydroxyproline.

According to Claim 15 of the present invention, in the biosensor as defined in Claim 1, the cell contraction agent is saccharide.

According to the present invention, the cellular components in the liquid solution are constricted, so that clogging by the cellular components in the liquid sample such as whole blood or a bacterial solution is avoided, thereby realizing a uniform permeation state. Therefore, a biosensor which is able to perform a more accurate measurement in a short time without inhibiting the reaction is realized. The saccharide as mentioned above includes a glucide such as glucose, scrose, and trehalose, as well as sugar alcohol such as glucitol.

According to Claim 16 of the present invention, in the biosensor as defined in Claim 1, the biosensor is a one-step immunochromatography test specimen.

According to the present invention, many measurement targets can be measured by obtaining antibodies or antigens for the measurement targets, and the applied sample solution is developed on the biosensor while being efficiently agitated by a turbulent blow generated by the retiform structure. Therefore, the permeability of a reactive layer carrier is enhanced, and a more uniform permeation is realized, resulting in a biosensor which enables a simple, quick, more sensitive, and higher-performance measurement.

The "one-step" as mentioned above represents the measurement operation which only requires the sample solution to be dropped to the test specimen without the need to preprocess the sample solution, and does not require a developing solution to develop the sample solution after the dropping, nor require a washing process. Further, the immunochromatography test specimen as mentioned above is a sensor for detecting an analyte in the sample solution on a carrier where chromatography development is performed, by utilizing an antigen-antibody reaction.

According to Claim 17 of the present invention, there is provided a method for analyzing a constituent of blood by employing a biosensor made of a dried porous material. The biosensor comprises: a sample introductory part for introducing a sample solution; and a developing layer for developing the sample solution, and in this biosensor the developing layer includes: a marker reagent holding part where a marker reagent is held in a dry state so that it can be eluted by the development of the sample solution; and a reagent immobilization part where a reagent which can bind to an analyte and is involved in a reaction is immobilized so that it is not eluted, and when the sample solution is introduced to the sample introductory part, the sample solution permeates the developing layer to reach the marker reagent holding part, and moves to the reagent immobilization part while eluting the marker reagent, whereby the reaction among the analyte, the marker reagent, and the immobilized reagent occurs. The analysis method comprises: measuring an amount of marker reagent bound in the reagent immobilization part, thereby qualitatively or quantitatively measuring the analyte included in the sample solution, and in this analysis method a retiform structure is arranged in the sample introductory part, and the blood is developed.

According to the present invention, the applied sample solution is developed on the biosensor while being agitated by a turbulent flow caused by the retiform structure, whereby the permeability of a reactive layer carrier is enhanced, and a more uniform permeation is realized, resulting in a blood constituent analysis method which enables a simple, quick, more sensitive, and higher-performance measurement.

The retiform structure as mentioned above is formed by performing a molding process on a fiber or resin to reticulate the same, and it makes no difference whether the retiform structure itself has capillary activity or absorbability or not. The reticulum at this time may have any shape as long as it is polygonal, and the size thereof does not matter. It is preferable that meshes are regularly arranged. Further, this retiform structure is preferably a single layer.

Further, the turbulent flow is a flow in which a fluid irregularly moves in disorder and a stream line shows a fine and irregular fluctuation.

According to the present invention, there is provided a method for analyzing a constituent of blood by employing a biosensor made of a dried porous material, wherein the sample introductory part of said biosensor is provided with a space forming part for forming a space on the developing layer so as to produce a cavity into which the sample solution is sucked and, held therein.

According to the present invention, a specific amount of sample solution is sucked in the sample introductory part as the cavity formed by the space forming material, and the applied sample solution is developed on the biosensor while being efficiently agitated by a turbulent flow generated by the reriform tissue. Therefore, the permeability of a reactive layer carrier is enhanced, and a more uniform permeation is realized, resulting in a blood constituent analysis method which enables a simple, quick, more sensitive, and higher-performance measurement.

According to the method of present invention, a cell contraction agent holding part holding cell contraction agent which contracts cellular is included in the space forming part.

According to the present invention, there no need to previously remove the cellular components in the sample solution or fragmentizing the same, thereby realizing a biosensor which enables a simpler and quicker measurement.

According to the method of the present invention, the retiform structure comprises a single layer with a mesh having a pore size of 0.1 mm to 2 mm arranged in the sample introductory part.

According to the present invention, clogging by the cellular component is avoided, and the sample solution is efficiently agitated by a turbulent flow generated by the retiform structure. Therefore, a smaller amount of sample solution realizes a uniform permeation on a reactive layer carrier, resulting in a biosensor which enables a simple, quick, sensitive, and high-performance measurement.

According to Claim 18 of the present invention, there is provided a blood constituent analysis method, wherein the biosensor comprises the marker reagent holding part in the cavity, and the sample solution is introduced to the sample introductory part, the sample solution is developed in the developing layer while the marker reagent is eluted to reach the reagent immobilization part, thereby occurring a reaction among the analyte, the marker reagent, and the immobilized reagent.

According to the present invention, a specific amount of sample solution is sucked in the sample introductory part as the cavity formed by the space forming material, and the applied sample solution is developed on the biosensor while being efficiently agitated by a turbulent flow generated by the retiform tissue and, thus, more thoroughly reacting with the marker reagent. Therefore, the permeability of a reactive layer carrier is enhanced, and a more uniform permeation is realized, resulting in a blood constituent analysis method which enables a simple, quick, more sensitive, and higher-performance measurement.

According to Claim 19 of the present invention, in the blood constituent analysis method as defined in Claim 17, the retiform structure is arranged at the end part of the sample introductory part.

According to the present invention, as soon as the sample is applied, the applied sample solution is developed on the biosensor while being efficiently agitated by a turbulent flow generated by the retiform structure, whereby a more uniform and effective cellular constriction is performed, and the sample solution permeates a reactive layer carrier uniformly, resulting in a blood constituent analysis method which enables a simple, quick, more sensitive, and higher-performance measurement.

According to Claim 20 of the present invention, in the blood constituent analysis method as defined in Claim 17, the retiform structure and the cell contraction agent holding part are arranged so that the edges of the end parts thereof are kept aligned.

According to the present invention, the introduced sample solution can immediately react with the cell contraction agent as well as receive the effect of turbulent flow at the introduction, whereby the sample solution can efficiently react with the cell contraction agent and is developed on the biosensor with cells therein constricted more uniformly and effectively. Therefore, the sample solution permeates a reactive layer carrier uniformly, resulting in a blood constituent analysis method which enables a simple, quick, more sensitive, and higher-performance measurement.

According to Claim 21 of the present invention, in the blood constituent analysis method as defined in Claim 17, a space enabling the sample solution to flow therein is arranged between the retiform structure and the cell contraction agent holding part.

According to the present invention, the sample solution is smoothly introduced, and a sufficient constriction reaction which occurs by means of the cell contraction agent and effect of turbulent flow due to the retiform structure can be obtained, whereby the sample solution is developed on the biosensor with cells therein constricted more uniformly and effectively. Therefore, the sample solution permeates a reactive layer carrier uniformly, resulting in a blood constituent analysis method which enables a simple, quick, more sensitive, and higher-performance measurement.

According to Claim 22 of the present invention, in the blood constituent analysis method as defined in Claim 17, the retiform structure is arranged so that warp threads thereof are parallel to the direction in which the sample is developed on the developing layer.

According to the present invention, the sample solution which is developed while being efficiently agitated by a turbulent flow is efficiently led in the direction of the development by a capillary phenomenon caused by warp threads of the retiform structure that extend in the direction of the sample development. Therefore, the sample solution permeates a reactive layer carrier uniformly, resulting in a blood constituent analysis method which enables a simple, quick, more sensitive, and higher-performance measurement.

The warp thread as mentioned above is a part of the retiform structure in which a molded product of a fiber or resin forming the reticulum extends in the direction of the development. It is preferable that the warp thread is regularly arranged so that it is parallel to the direction of the development. Directions of tissues extending in other directions than that described above do not matter.

According to Claim 23 of the present invention, in the blood constituent analysis method as defined in Claim 17, the reitform tissue is made of synthetic resin.

According to the present invention, the retiform structure itself lacks the water retaining capacity, whereby the sample solution is well drained, and the applied sample solution is quickly developed on the biosensor while being efficiently agitated by a turbulent flow generated by the retiform structure. Therefore, a smaller amount of sample solution realizes a uniform permeation on a reactive layer carrier, resulting in a blood constituent analysis method which enables a simple, quick, sensitive, and high-performance measurement.

According to Claim 24 of the present invention, in the blood constituent analysis method as defined in Claim 17, the retiform structure is made of a chemical fiber such as polyester.

According to the present invention, the retiform structure itself lacks the water retaining capacity, whereby the sample solution is well drained, and the applied sample solution is efficiently and quickly developed on the biosensor by a capillary phenomenon, while being efficiently agitated by a turbulent flow generated by the retiform structure. Therefore, a smaller amount of sample solution realizes a uniform permeation on a reactive layer carrier, resulting in a blood constituent analysis method which enables a simple, quick, more sensitive, and higher-performance measurement.

Further, the retiform structure as mentioned above is formed by performing a welding process such as a thermocompression bonding and a press work on a chemical fiber, to reticulate the same.

According to Claim 25 of the present invention, in the blood constituent analysis method as defined in Claim 17, the retiform structure is a fabric obtained by weaving the chemical fiber such as polyester.

According to the present invention, the retiform structure itself lacks the water retaining capacity, whereby the sample solution is well drained, and the applied sample solution is efficiently and quickly developed on the biosensor by a capillary phenomenon, while being efficiently agitated by a turbulent flow generated by the retiform structure. Therefore, a smaller amount of sample solution realizes a uniform permeation on a reactive layer carrier, resulting in a blood constituent analysis method which enables a simple, quick, more sensitive, and higher-performance measurement.

Further, the retiform structure as mentioned above is a fabric or a knit formed by the process of weaving or knitting a chemical fiber.

According to Claim 26 of the present invention, in the blood constituent analysis method as defined in Claim 17, the retiform structure is treated with a surfactant so that it can permeate.

According to the present invention, the retiform structure does not repel the liquid sample, and the applied sample solution is quickly developed on the biosensor while being efficiently agitated by a turbulent flow generated by the retiform structure. Therefore, a smaller amount of sample solution realizes a uniform permeation on a reactive layer carrier, resulting in a blood constituent analysis method which enables a simple, quick, sensitive, and high-performance measurement.

According to Claim 27 of the present invention, in the blood constituent analysis method as defined in Claim 17, the sample solution to be applied is whole blood.

According to the present invention, there is no need to previously subject blood to some processing, thereby realizing a blood constituent analysis method enabling a simple, quick, sensitive, and high-performance measurement, by which a safer and more sanitary blood examination can be conducted.

According to Claim 28 of the present invention, in the blood constituent analysis method as defined in Claim 17, the cell contraction agent is inorganic salt.

According to the present invention, the cellular components in the liquid solution are constricted, so that clogging by the cellular components in the liquid sample such as whole blood or a bacterial solution is avoided, thereby realizing a uniform permeation state. Therefore, a blood constituent analysis method by which a more accurate measurement can be performed in a short time without inhibiting the reaction is realized. The inorganic salt as mentioned above is an inorganic compound including salt, such as sodium chloride, potassium chloride, and sodium phosphate.

According to Claim 29 of the present invention, in the blood constituent analysis method as defined in Claim 17 the cell contraction agent is amino acid.

According to the present invention, the cellular components in the liquid solution are constricted, so that clogging by the cellular components in whole blood is avoided, thereby realizing a uniform permeation state. Therefore, a blood constituent analysis method by which a more accurate measurement can be performed in a short time without inhibiting the reaction is realized. The amino acid as mentioned above is a compound which has a carboxyl group and an amino group in an identical molecule, such as glycin and glutamic acid, and further includes imino acid such as proline and hydroxyproline.

According to Claim 30 of the present invention, in the blood constituent analysis method as defined in Claim 17, the cell contraction agent is saccharide.

According to the present invention, the cellular components in the liquid solution are constricted, so that clogging by the cellular components in whole blood is avoided, thereby realizing a uniform permeation state. Therefore, a blood constituent analysis method by which a more accurate measurement can be performed in a short time without inhibiting the reaction is realized. The saccharide as mentioned above includes a glucide such as glucose, scrose, and trehalose, as well as sugar alcohol such as glucitol.

According to Claim 31 of the present invention, in the blood constituent analysis method as defined in Claim 17, the biosensor is a one-step immunochromatography test specimen.

According to the present invention, many measurement targets can be measured by obtaining antibodies or antigens for the measurement targets, and the sample solution to be applied is subjected to no processing and no means is required after the sample application until the reaction end. The applied sample solution is developed on the biosensor while being efficiently agitated by a turbulent blow generated by the retiform structure. Therefore, the permeability of a reactive layer carrier is enhanced, and a more uniform permeation is realized, resulting in a blood constituent analysis method which enables a simple, quick, more sensitive, and higher-performance measurement.

Further, the "one-step" as mentioned above represents the measurement operation which only requires the sample solution to be dropped to the test specimen without the need to preprocess the sample solution, and does not require a developing solution to develop the sample solution after the dropping, nor require a washing process. Further, the immunochromatography test specimen as mentioned above is a sensor for detecting an analyte in the sample solution on a carrier where chromatography development is performed, by utilizing an antigen-antibody reaction.

As described above, according to the present invention, it is possible to realize a simple, quick, sensitive, and high-performance biosensor which requires no operation of previously removing the cellular components from the specimen including the cellular components, generates no clogging on the carrier by the cellular components, and has an enhanced permeability of the reactive layer carrier, and a blood constituent analysis method employing the biosensor.

### BRIEF DESCRIPTION OF DRAWINGS

Figure 1 is a diagram of a biosensor according to a first embodiment of the present invention.
Figure 2 is a diagram of a biosensor according to a second embodiment of the present invention.
Figure 3 is a diagram of the biosensor according to the second embodiment of the invention.
Figure 4 is a diagram of the biosensor according to the second embodiment of the invention.
Figure 5 is a diagram illustrating a quantitative performance of the biosensor in a case where a retiform structure is not employed as an example.
Figure 6 is a diagram illustrating a quantitative performance in a case where a biosensor having a retiform structure according to the present invention is employed as an example.
Figure 7 is a top view of a retiform structure according to the present invention.
Figure 8 is a side view of the retiform structure shown in figure 7.

### BEST MODE TO EXECUTE THE INVENTION

Hereinafter, embodiments according to the present invention will be described with reference to the drawings. The embodiments described here are given only as examples and the present invention is not restricted to these embodiments.

### (Embodiment 1)

A first embodiment of the present invention will be described with reference to the drawing.

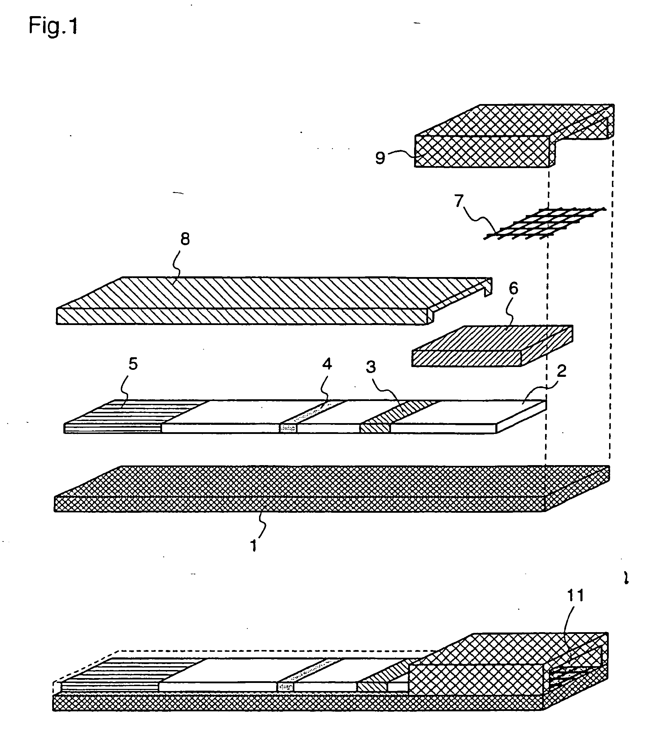

Figure 1 is a diagram illustrating a biosensor for performing a chromatography measurement according to the first embodiment. In figure 1, numeral 1 denotes a reactive layer carrier support for supporting a chromatography material, which is made of plastic or the like. Numeral 2 denotes a developing layer for developing a sample solution, which is made of nitrocellulose or the like, numeral 3 denotes a marker reagent holding part where a dissoluble marker reagent is held so as to permeate the developing layer, numeral 4 denotes a sample immobilization part as an area on the developing layer 2 where a sample such as a specific protein is immobilized, numeral 5 denotes a water absorbing part for finally absorbing the sample solution, numeral 6 denotes a sample holding part for temporarily holding the applied sample, which is made of a - nonwoven fabric, glass fiber filter paper or the like having high hydrophilia, numeral 7 denotes a retiform structure, numeral 8 denotes a liquid-impermeable sheet, numeral 9 denotes a material for forming a space in which the sample solution is sucked to be held, and numeral 11 denotes a sample introductory part where the sample solution is applied or sucked. The respective regions 2∼11 are formed on the reactive layer carrier support 1. Further, the sample holding part 6 may hold a cellular component shrinker.

Next, an operation of the biosensor according to the first embodiment will be described.

On the biosensor shown in figure 1, when a sample solution is applied to the sample introductory part 11, the sample solution reaches the area of the sample holding part 6, while being efficiently agitated by a turbulent flow caused by the retiform structure 7. The shrinker held in the area 6 is dissolved by permeation of the sample solution and causes cellular components to constrict, and the sample solution in which the constricted cellular components are mixed permeates the developing layer 2 and reaches the area of the marker reagent holding part 3. Then, the marker reagent held in the area of the marker reagent holding part 3 is dissolved by permeation of the sample solution, and is developed to reach the sample immobilization part 4. In the sample immobilization part 4, a binding reaction among the marker reagent dissolved from the area of the marker reagent holding part 3, an analysis target in the liquid sample, and the immobilized reagent occurs. At this time, when the analysis target exists in the liquid sample, some color reaction is seen in the area of the sample immobilization part 4. Finally, the sample solution is absorbed in the water absorbing part 5, thereby ending the reaction.

Since the retiform structure 7 is provided on the biosensor shown in figure 1, the cellular components in the sample solution reaches the sample holding part while being agitated. At this time, the dissolving cell contraction agent sufficiently reacts with the sample solution in a cavity formed by the space forming material 9 through the influence of the retiform structure. Accordingly, the cellular components in the sample solution constrict with the elution of the shrinker. Therefore, the sample solution can pass on the developing layer without causing clogging, and smoothly permeates the biosensor in a state where the cellular components are mixed therein.

As described above, according to the first embodiment, the cellular components in the sample solution efficiently constrict through the influence of the retiform structure included in the biosensor, and the sample solution quickly permeates the developing layer without causing clogging in a state where the cellular components are mixed therein, thereby realizing a simpler, quicker, and higher-performance chromatography measurement without previously separating the cellular components such as blood cells.

### (Embodiment 2)

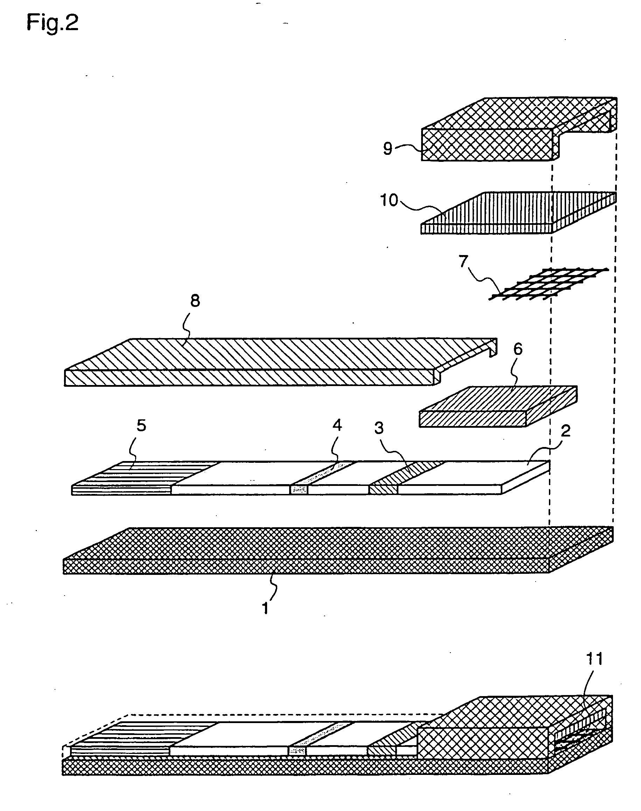

Hereinafter, a second embodiment of the present invention will be described with reference to figures 2, 3 and 4.

Figure 2 is a diagram illustrating a biosensor for performing a chromatography measurement according to the second embodiment. In figure 2, numeral 1 denotes a reactive layer carrier support for supporting a chromatography material, which is made of plastic or the like. Numeral 2 denotes a developing layer for developing a sample solution, which is made of nitrocellulose or the like, numeral 3 denotes a marker reagent holding part where a dissoluble marker reagent is held so as to permeate the developing layer, numeral 4 denotes a sample immobilization part as an area on the developing layer 2 where a sample such as a specific protein is immobilized, numeral 5 denotes a water absorbing part for finally absorbing the sample solution, numeral 6 denotes a sample holding part for temporarily holding an applied sample, which is made of a nonwoven fabric, glass fiber filter paper or the like having high hydrophilia, numeral 7 denotes a retiform structure, numeral 8 denotes a liquid-impermeable sheet, numeral 9 denotes a material for forming a space in which the sample solution is sucked to be held, numeral 10 denotes a cell contraction agent holding part arranged between the space forming material 9 and the retiform structure 7, and numeral 11 denotes a sample introductory part where the sample solution is applied or sucked. The respective regions 2∼11 are formed on the reactive layer carrier support 1.

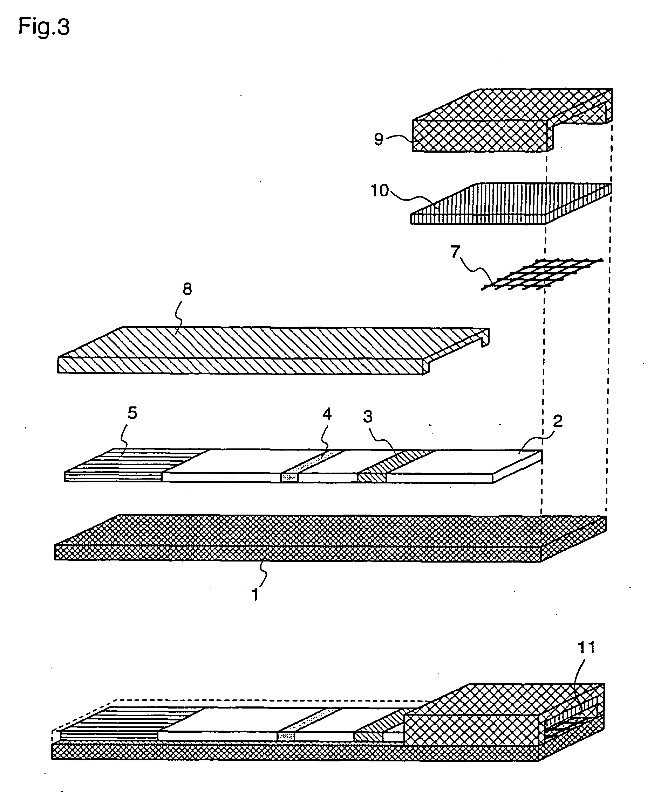

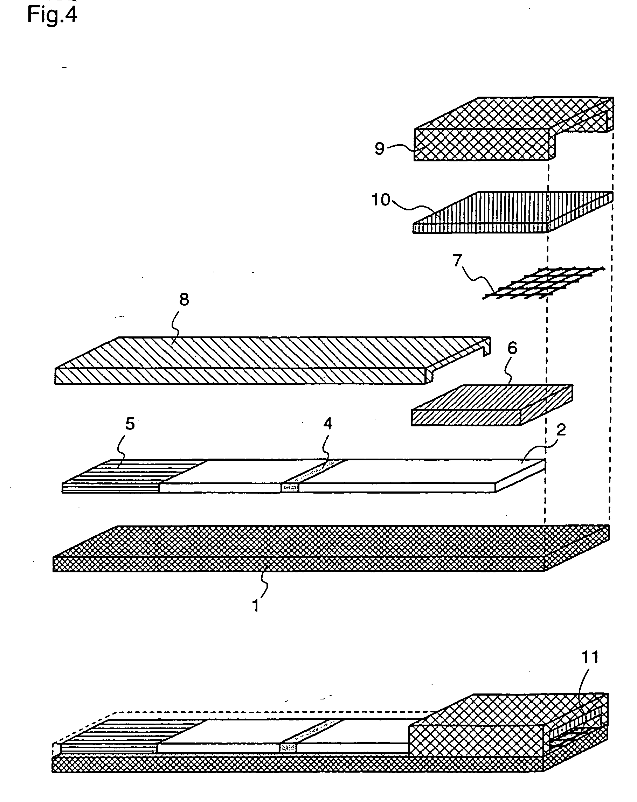

Figure 3 illustrates the construction which is different from that shown in figure 2 in that the sample holding part 6 is removed, and a sample solution is held in a cavity formed by the space forming material 9. Further, figure 4 illustrates the construction which is different from that shown in figure 2 in that the marker reagent holding part 3 is removed, and the sample holding part 6 or the cell contraction agent holding part 10 also serves as the marker reagent holding part.

Next, an operation of the biosensor according to the second embodiment will be described.

On each of the biosensors shown in figures 2, 3, and 4, when a sample solution is applied to the sample introductory part 11, the sample solution reaches the area of the sample holding part 6, while being efficiently agitated by a turbulent flow caused by the retiform structure 7. The shrinker held in the area 6 is dissolved by permeation of the sample solution and causes cellular components to constrict, and the sample solution in which the constricted cellular components are mixed permeates the developing layer 2 and reaches the area of the marker reagent holding part 3. Then, the marker reagent held in the area of the marker reagent holding part 3 is dissolved by permeation of the sample solution, and is developed to reach the sample immobilization part 4. In the sample immobilization part 4, a binding reaction among the marker reagent dissolved from the area of the marker reagent holding part 3, an analysis target in the liquid sample, and the immobilized reagent occurs.

At this time, when the analysis target solution exists in the liquid sample, some color reaction is seen in the area of the sample immobilization part 4. Finally, the sample solution is absorbed in the water absorbing part 5, thereby ending the reaction.

On the biocensor shown in figure 2, the cell contraction agent holding part 10 is provided between the space forming material 9 and the retiform structure 7, and the edges of the end parts thereof are kept aligned. Thus, the moment when the sample solution is dropped, the cellular components in the liquid sample are constricted by the cell contraction agent. At this time, since the retiform structure 7 is arranged on the sample holding part 6, the sample solution efficiently reacts with the cell contraction agent while being agitated due to the retiform structure, and reaches the sample holding part. Therefore, the sample solution can permeate the developing layer without causing clogging, and smoothly permeate the biosensor in a state where the cellular components are mixed. Further, since there is no need for the reaction between the cellular components in the sample solution and the cell contraction agent in the sample holding part 6, the sample holding part 6 is not clogged by the cellular components. Therefore, there is no necessity to consider a material, a fiber density, or a pore size of the sample holding part 6, thereby expanding the scope of selection.

The biosensor shown in figure 3 is different from that shown in figure 2 in that the sample holding part 6 is removed, and the sample solution is held in the cavity formed by the space forming material 9. In this case, the sample solution efficiently reacts with the cell contraction agent while being agitated through the influence of the retiform structure 7. Further, there is no loss of sample solution that is caused by absorption in the sample holding part 6, whereby a measurement can be performed with a smaller amount of sample solution to be applied.

Further, the biosensor shown in figure 4 is different from that shown in figure 2 in that the marker reagent holding part 3 is removed, and the sample holding part 6 or the cell contraction agent holding part 10 also serves as the marker reagent holding part 3. In this case, the cell constriction reaction and the reaction between the analysis target and the marker reagent occur as soon as the sample is applied, whereby the reactions occur efficiently, and the time required for the reactions can be reduced.

As described above, according to the second embodiment, through the influence of the retiform structure included in the biosensor, the cellular components in the sample solution efficiently constrict, and the sample solution quickly permeates the developing layer without causing clogging in a state where the cellular components are mixed therein, thereby realizing a simple, quick, and high-performance chromatography measurement with a smaller amount of sample solution without previously separating the cellular components such as blood cells.

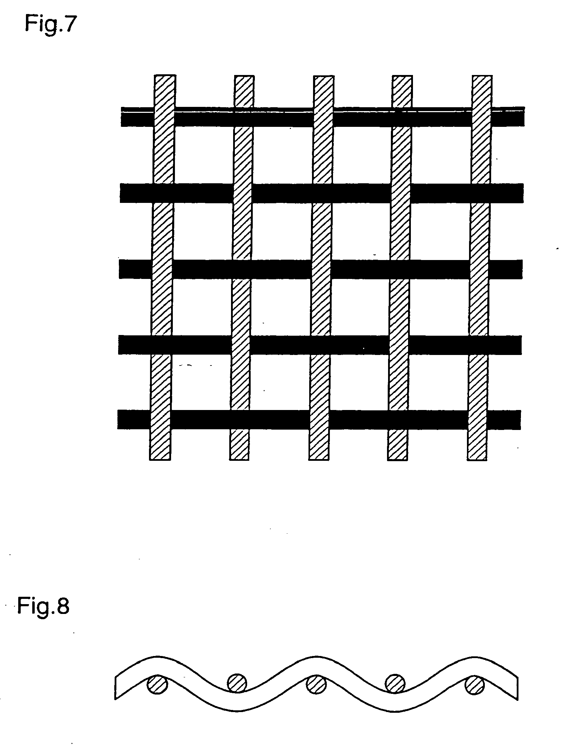

Figure 7 is a top view of the retiform structure, and figure 8 is a side view thereof. As seen in figures 7 and 8, meshes are regularly arranged. When a retiform structure composed of a single layer is employed, unevenness of the retiform structure generates a turbulent flow in the sample solution. This turbulent flow has an effect on the agitation between the sample solution and the cell contraction agent, resulting in a more efficient cell constriction reaction.

As a biosensor according to the present invention, one which is made of a chromatography material composed of arbitrary porous carriers such as nitrocellulose and a nonwoven fabric or glass fiber filter paper is employed. The biosensor made of such material has the function of analytically detecting a specific material employing an arbitrary principle of measurement such as an antigen-antibody reaction, thereby qualitatively or quantitatively measuring the material. Further, the description has been given of the embodiments taking the case where the antigen-antibody reaction employing the marker reagent occurs as an example, anything may be employed as long as it produces some change after the reaction, such as an enzyme.

According to the above-described embodiments, a more uniform permeation is realized, and thus it is possible to realize a simple, quick, sensitive, and high-performance chromatography measurement employing a small amount of specimen without the need to previously remove the cellular components.

### (Example)

A method for implementing the present invention will be described in more detail through a following example. The present invention is not restricted to the following example.

### (Example 1)

### (Quantitative analysis of CRP in blood)

A biosensor made of an immunochromatography which includes an anti-CRP antibody A immobilization line and a broad band of a marker reagent composed of a complex of an anti-CRP antibody B and gold colloid on a nitrocellulose film is manufactured. In the drawing, the test specimen includes the antibody immobilization part 4, the marker reagent holding part 3 as an area where the marker reagent which is the complex of an anti-CRP antibody B and gold colloid is held, the sample holding part 6, the retiform structure 7, and the space forming material 9 supporting the cell contraction agent holding part 10. - This biosensor is manufactured as follows.

### a) Preparation of biosensor

An anti-CRP antibody A solution which was diluted with a phosphate buffer solution to control the concentration was prepared. This antibody solution was applied on the nitrocellulose film by employing a solution discharge device. Thereby, a detecting antibody immobilization line was obtained on the nitrocellulose film. After being dried, this nitrocellulose film was immersed in a Tris-HCl buffer solution including 1% skim milk and shaken gently for 30 minutes. 30 minutes later, the film was moved into a Tris-HCl buffer solution tank, shaken gently for 10 minutes, and thereafter shaken gently in another Tris-HCl buffer solution tank for another 10 minutes, to wash the film. After washed twice, the film was taken out from the solution tank, and dried at room temperatures.

The gold colloid was prepared by adding 1% citric acid solution to a refluxing 100°C-solution of 0.01% chloroauric acid. After the reflux was continued for 30 minutes, it was cooled by being left at room temperatures. The anti-CRP antibody B was added to gold colloid solution which was prepared to pH9 by using 0.2M potassium carbonate solution, then the obtained solution was stirred for several minutes, and then 10% BSA (bovine serum albumin) solution of pH9 was added thereto by such an amount that 1% solution was finally obtained and stirred. Thereby, an antibody-gold colloid complex (marker antibody) was prepared. The marker antibody solution was centrifuged at 4°C and 20000G for 50 minutes, whereby the marker antibody was isolated. The isolated marker antibody was suspended in a washing buffer solution (1% BSA·phosphate buffer solution) and thereafter further centrifuged under the above-described condition to wash and isolate the marker antibody. The marker antibody was suspended in a certain amount of washing buffer solution and filtrated through a 0.8µm filter, thereby obtaining a marker antibody solution. The obtained marker antibody solution was prepared one-tenth as much as the gold colloid solution before being centrifuged, and stored at 4°C.

The marker antibody solution was set in the solution discharge device and applied to a position on an anti-CRP antibody A immobilization dry film, apart from an antibody immobilization position, and thereafter the film was dried. Thereby, the marker reagent holding part was obtained on the immobilization film.

The space forming material created by laminating a transparent PET of 100µm thick was affixed to the film, thereby forming a cavity (5.0mm wide×12.0mm long×0.5mm high). A potassium chloride solution prepared to 1.5M was dropped to the film, and thereafter the obtained film was immediately frozen with liquid nitrogen to be freeze-dried. Thereby, the space forming material having the shrinker holding part where the potassium chloride was impregnated was obtained.

The antibody immobilization film including the marker reagent holding region prepared as described above was affixed on the reactive layer carrier support made of a while PET of 0.5mm thick, the retifom tissue (a polyester mesh sheet), a nonwoven fabric as the sample holding part, and glass fiber filter paper as the water absorbing part were added thereto, and thereafter the film was cut into small pieces of 0.5cm wide. After the cutting, the sample introductory part is affixed to the cut film, thereby manufacturing an immunochromatography test specimen. This is employed as a biosensor.

### b) Preparation of sample

Human blood to which EDTA was added as an anticoagulant was prepared to have a hematocrit value of 45%. The CRP solutions of known concentrations were added to the blood, thereby preparing the CRP containing blood of various known concentrations.

### c) Measurement of the degree of coloration on test specimen

On the immunochromatography test specimen, approximately 50µl of whole blood including CRP was applied to the sample introductory part and developed in the direction of the water absorbing part, so that an antigen-antibody occurs, whereby a color reaction in the antibody immobilization part occurred. The coloration state 5 minutes after the sample application to the immunochromatography test specimen was measured by employing a reflective spectrophotometer (CS9300; Shimadzu Corporation made), and the coloration degree was computed.

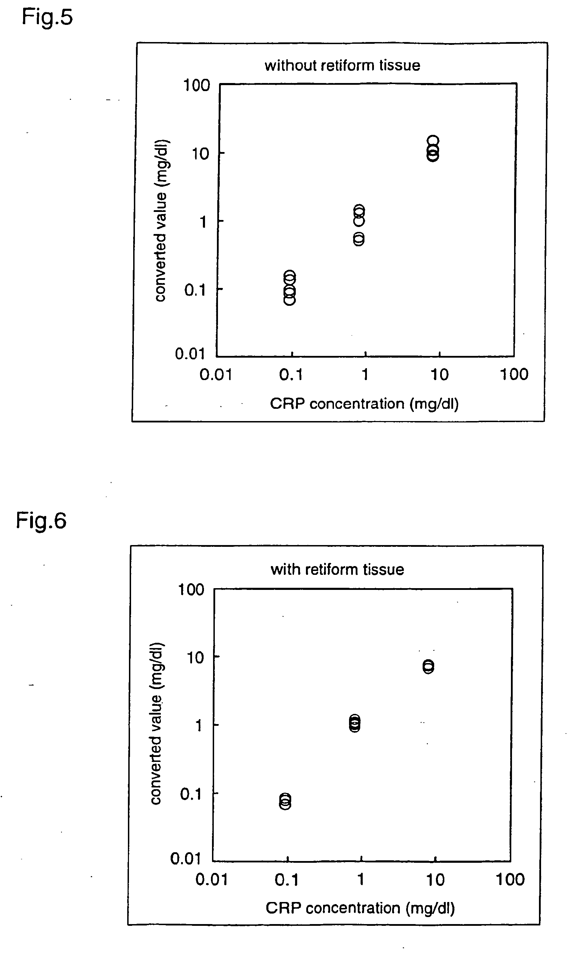

Whole blood including CRP of 0, 0.1, 1, and 10mg/dl were applied to the immunochromatography test specimen to be developed. The coloration state of the judgement part on the immunochromatography test specimen for whole blood of each CRP concentration was measured by the reflective spectrophotometer. An absorbance at 635nm was measured, and substituted into a previously formed calibration curve indicating a relationship between the CRP concentration and the absorbance. The result is shown in figures 5 and 6. Essentially, when for example the absorbance in the case of whole blood including CRP of 1mg/dl was measured, and the measured absorbance was substituted into the calibration curve, the CRP concentration should be 1mg/dl. However, actually the CRP concentration slightly deviates from 1mg/dl. The accuracy of measurement can be known by the amount of the deviation.

Figures 5 and 6 are diagrams illustrating quantitative performances, figure 5 showing a case where the retiform structure 7 of the biosensor shown in figure 2 is not employed and figure 6 showing a case where the retiform structure 7 is employed as shown in figure 2. The abscissa represents the CRP concentration of a sample applied to the biosensor. The ordinate represents the converted value of the antigen concentration obtained by substituting the signal in the color area on the test specimen into the calibration curve.

In the case where a biosensor which does not include the retiform structure is employed (figure 5), a CV value (coefficient of variation) ranges from 20 to 50% having a wide range of variations, which represents a low quantitative performance. On the other hand, in the case where a biosensor including the retiform structure is employed (figure 6), the CV value in each case is amazingly within 3 to 8%, which represents a sufficiently increased quantitative performance. From the above results, it can be understood that the arrangement of the retiform structure on the biosensor greatly concerns the enhancement in the quantitative performance.

As a biosensor according to the embodiments of the present invention, the immunochromatography test specimen which is made of a chromatography material composed of arbitrary porous carriers such as nitrocellulose and glass fiber filter paper is employed. The biosensor made of such material has the function of analytically detecting a specific material employing an arbitrary principle of measurement such as an antigen-antibody reaction, thereby qualitatively or quantitatively measuring the material.

While in this example the immunochromatography test specimen which is provided with the marker reagent holding part and the reagent immobilization part on an identical nitrocellulose film is employed, the marker reagent holding part supporting a marker reagent may be arranged on a porous carrier made of a material different from nitrocellulose, such as a nonwoven fabric, on a support body. While gold colloid is employed as a marker composing the marker reagent, anything that produces some change after the reaction may be employed, such as a coloring material, a fluorescent material, a phosphorescent material, a light-emitting material, an oxidation-reduction material, an enzyme, a nucleic acid, and an endoplasmic reticulum. Further, the water absorbing part may be excluded from the constituent members.

As sample solutions to be measured, there are for example water, an aqueous solution, bodily fluids such as urine, blood, blood plasma, blood serum, and saliva, a solution in which a solid, fine particles, or gas is dissolved, and the like. Applications thereof include a urinalysis, a pregnancy test, a water examination, a fecal examination, soil analysis, food analysis and the like. While the description has been given of the example taking the C-reactive protein (CRP) as an example of the analyte, it may be an antibody, immunoglobulin, hormone, a protein and a protein derivative such as an enzyme and peptide, a bacterium, a virus, the true fungi, mycoplasma, a parasite and a infectious material such as a product and a component thereof, a chemical drug such as a curative medicine and a drug of abuse, or a tumor marker. Specifically, the analyte may be for example human chrionic gonadotropin (hCG), luteinizing hormone (LH), thyroid-stimulating hormone, follicular hormone, parathyroid hormone, adrenocorticotropic hormone, estradiol, prostatic specific antigen, Hepatitis B surface antigen, myoglobin, CRP, cardiac troponin, HbA1c, albumin or the like.

According to the mode as described above, the permeability of the reactive layer carrier is enhanced, and a more uniform permeation is realized, thereby realizing a simple, quick, more sensitive, and higher-performance chromatography measurement.

### APPLICABILITY IN INDUSTRY

As described above, a biosensor according to the present invention is suited to enable a measurement with a small amount of specimen, for which whole blood with a reduced influence of blood cell can be used without previously separating blood plasma components from the blood.

## Claims

1. A biosensor made of a dried porous material,
said biosensor comprising : a sample introductory part for introducing a sample solution; and a developing layer for developing the sample solution, wherein
the developing layer includes: a marker reagent holding part where a marker reagent is held in a dry state so that it can be eluted by the development of the sample solution; and a reagent immobilization part where a reagent which can bind to an analyte and is involved in a reaction is immobilized so that it is not eluted, and when the sample solution is introduced to the sample introductory part, the sample solution permeates the developing layer to reach the marker reagent holding part, and moves to the reagent immobilization part while eluting the marker reagent, whereby the reaction among the analyte, the marker reagent, and the immobilized reagent occurs, and
an amount of marker reagent bound in the reagent immobilization part is measured, thereby qualitatively or quantitatively measuring the analyte included in the sample solution, wherein
the sample introductory part is provided with a space forming part for forming a space on the developing layer so as to produce a cavity into which the sample solution is sucked and held therein,
a cell contraction agent holding part holding cell contraction agent which contracts cellular components is included in the space forming part; and
a retiform structure comprising a single layer with a mesh having a pore size of 0.1 mm to 2 mm is arranged in the sample introductory part.

2. The biosensor as defined in claim 1, wherein
the marker reagent holding part is disposed in the cavity, and
the sample solution is introduced to the sample introductory part, the sample solution is developed in the developing layer while the marker reagent is eluted to reach the reagent immobilization part, thereby occurring a reaction among the analyte, the marker reagent, and the immobilized reagent.

3. The biosensor as defined in claim 1, wherein
the retiform structure is arranged at the end part of the sample introductory part.

4. The biosensor as defined in claim 1, wherein
the retiform structure and the cell contraction agent holding part are arranged so that the edges of the end parts thereof are kept aligned.

5. The biosensor as defined in claim 1, wherein
a space enabling the sample solution to flow therein is arranged between the retiform structure and the cell contraction agent holding part.

6. The biosensor as defined in claim 1, wherein
the retiform structure is arranged so that warp threads thereof are parallel to the direction in which the sample is developed on the developing layer.

7. The biosensor as defined in claim 1, wherein
the retiform tissue is made of synthetic resin.

8. The biosensor as defined in claim 1, wherein
the retiform structure is made of a chemical fiber such as polyester.

9. The biosensor as defined in claim 1, wherein
the retiform structure is a fabric obtained by weaving the chemical fiber such as polyester.

10. The biosensor as defined in claim 1, wherein
the retiform structure is treated with a surfactant so that it can permeate.

11. The biosensor as defined in claim 1, wherein
the sample solution to be applied is blood.

12. The biosensor as defined in claim 1, wherein
the sample solution to be applied is a solution including bacteria.

13. The biosensor as defined in claim 1, wherein
the cell contraction agent is inorganic salt.

14. The biosensor as defined in claim 1, wherein
the cell contraction agent is amino acid.

15. The biosensor as defined in claim 1, wherein
the cell contraction agent is saccharide.

16. The biosensor as defined in claim 1, wherein
the biosensor is a one-step immunochromatography test specimen.

17. A method for analyzing a constituent of blood by employing a biosensor made of a dried porous material,
said biosensor comprising : a sample introductory part for introducing a sample solution; and a developing layer for developing the sample solution, wherein
the developing layer includes : a marker reagent holding part where a marker reagent is held in a dry state so that it can be eluted by the development of the sample solution; and a reagent immobilization part where a reagent which can bind to an analyte and is involved in a reaction is immobilized so that it is not eluted, and when the sample solution is introduced to the sample introductory part, the sample solution permeates the developing layer to reach the marker reagent holding part, and moves to the reagent immobilization part while eluting the marker reagent, whereby the reaction among the analyte, the marker reagent, and the immobilized reagent occurs, and
said analysis method comprising: measuring an amount of marker reagent bound in the reagent immobilization part, thereby qualitatively or quantitatively measuring the analyte included in the sample solution, wherein
the sample introductory part is provided with a space forming part for forming a space on the developing layer so as to produce a cavity into which the sample solution is sucked and held therein,
a cell contraction agent holding part holding cell contraction agent which contracts cellular components is included in the space forming part; and
a retiform structure comprising a single layer with a mesh having a pore size of 0.1 mm to 2 mm is arranged in the sample introductory part, and the blood is developed.

18. The blood constituent analysis method as defined in claim 17, wherein
the biosensor comprises the marker reagent holding part in the cavity, and
the sample solution is introduced to the sample introductory part, the sample solution is developed in the developing layer while the marker reagent is eluted to reach the reagent immobilization part, thereby occurring a reaction among the analyte, the marker reagent, and the immobilized reagent.

19. The blood constituent analysis method as defined in claim 17, wherein
the retiform structure is arranged at the end part of the sample introductory part.

20. The blood constituent analysis method as defined in claim 17, wherein
the retiform structure and the cell contraction agent holding part are arranged so that the edges of the end parts thereof are kept aligned.

21. The blood constituent analysis method as defined in claim 17, wherein
a space enabling the sample solution to flow therein is arranged between the retiform structure and the cell contraction agent holding part.

22. The blood constituent analysis method as defined in claim 17, wherein
the retiform structure is arranged so that warp threads thereof are parallel to the direction in which the sample is developed on the developing layer.

23. The blood constituent analysis method as defined in claim 17, wherein
the retiform tissue is made of synthetic resin.

24. The blood constituent analysis method as defined in claim 17, wherein
the retiform structure is made of a chemical fiber such as polyester.

25. The blood constituent analysis method as defined in claim 17, wherein
the retiform structure is a fabric obtained by weaving the chemical fiber such as polyester.

26. The blood constituent analysis method as defined in claim 17, wherein
the retiform structure is treated with a surfactant so that it can permeate.

27. The blood constituent analysis method as defined in claim 17, wherein
the sample solution to be applied is whole blood.

28. The blood constituent analysis method as defined in claim 17, wherein
the cell contraction agent is inorganic salt.

29. The blood constituent analysis method as defined in claim 17, wherein
the cell contraction agent is amino acid.

30. The blood constituent analysis method as defined in claim 17, wherein
the cell contraction agent is saccharide.

31. The blood constituent analysis method as defined in claim 17, wherein
the biosensor is a one-step immunochromatography test specimen.

## Patentansprüche

1. Biosensor aus getrocknetem porösen Material,
der Biosensor umfassend: ein Probeneinführungsteil zum Einführen einer Probenlösung; und eine Entwicklungsschicht zum Entwickeln der Probenlösung, wobei
die Entwicklungsschicht Folgendes enthält: ein Markerreagenshalteteil, in dem ein Markerreagens in einem trockenen Zustand gehalten ist, sodass es durch die Entwicklung der Probenlösung eluiert werden kann; und ein Reagensfixierteil, in dem ein Ragens, das an einen Analyt binden kann und an einer Reaktion beteiligt ist, fixiert ist, sodass es nicht eluiert wird, und, wenn die Probenlösung in das Probeneinführungsteil eingeführt ist, die Probenlösung die Entwicklungsschicht zum Erreichen des Markerreagenshalteteil durchdringt und sich zu dem Reagensfixierteil bewegt, während sie das Markerreagens eluiert, wobei die Reaktion zwischen dem Analyt, dem Markerreagens und dem fixierten Reagens erfolgt, und
eine Markerreagensmenge, die in dem Reagensfixierteil gebunden ist, gemessen wird, wodurch der Analyt, der in der Probenlösung enthalten ist, qualitativ oder quantitativ gemessen wird, wobei
das Probeneinführungsteil mit einem raumbildenden Teil zum Ausbilden eines Raums auf der Entwicklungsschicht zum Hervorbringen eines Hohlraums versehen ist, in den die Probenlösung eingesaugt und darin gehalten wird; und
ein Zellenkontraktionswirkstoffhalteteil, das Zellenkontraktionswirkstoff hält, der Zellkomponenten zusammenzieht, in dem raumbildenden Teil enthalten ist; und
eine retikuläre Struktur, die eine einzelne Schicht mit einem Netz mit einer Porengröße von 0,1 mm bis 2 mm umfasst, in dem Probeneinführungsteil angeordnet ist.

2. Biosensor nach Anspruch 1, wobei das Markerreagenshalteteil in dem Hohlraum angeordnet ist, und
die Probenlösung in das Probeneinführungsteil eingeführt wird, die Probenlösung in der Entwicklungsschicht entwickelt wird, während das Markerreagens zum Erreichen des Reagensfixierteils eluiert wird, wodurch eine Reaktion zwischen dem Analyt, dem Markerreagens und dem fixierten Reagens erfolgt.

3. Biosensor nach Anspruch 1, wobei
die retikuläre Struktur am Endteil des Probeneinführungsteils angeordnet ist.

4. Biosensor nach Anspruch 1, wobei
die retikuläre Struktur und das Zellenkontraktionswirkstoffhalteteil derart angeordnet sind, dass die Kanten ihrer Endteile aneinander ausgerichtet gehalten sind.

5. Biosensor nach Anspruch 1, wobei
wobei ein Raum, der es der Probenlösung ermöglicht, darin zu fließen, zwischen der retikulären Struktur und dem Zellenkontraktionswirkstoffhalteteil angeordnet ist.

6. Biosensor nach Anspruch 1, wobei
die retikuläre Struktur derart angeordnet ist, dass Kettfäden davon parallel zu der Richtung verlaufen, in der die Probe auf der Entwicklungsschicht entwickelt wird.

7. Biosensor nach Anspruch 1, wobei
die retikuläre Struktur aus Kunstharz hergestellt ist.

8. Biosensor nach Anspruch 1, wobei
die retikuläre Struktur aus Chemiefaser, wie etwa Polyester, hergestellt ist.

9. Biosensor nach Anspruch 1, wobei
die retikuläre Struktur ein Gewebe ist, das durch Weben der Chemiefaser, wie etwa Polyester, erzielt ist.

10. Biosensor nach Anspruch 1, wobei
die retikuläre Struktur mit einem grenzflächenaktiven Stoff behandelt ist, sodass sie durchlässig ist.

11. Biosensor nach Anspruch 1, wobei
die Probenlösung, die angewendet werden soll, Blut ist.

12. Biosensor nach Anspruch 1, wobei
die Probenlösung, die angewendet werden soll, eine Lösung ist, die Bakterien enthält.

13. Biosensor nach Anspruch 1, wobei
der Zellenkontraktionswirkstoff anorganisches Salz ist.

14. Biosensor nach Anspruch 1, wobei
der Zellenkontraktionswirkstoff Aminosäure ist.

15. Biosensor nach Anspruch 1, wobei
der Zellenkontraktionswirkstoff Saccharid ist.

16. Biosensor nach Anspruch 1, wobei
der Biosensor ein immunchromatographischer Testprobekörper in einem Schritt ist.

17. Verfahren zum Analysieren eines Bestandteils von Blut durch Einsetzen eines Biosensors aus getrocknetem porösen Material,
der Biosensor umfassend: ein Probeneinführungsteil zum Einführen einer Probenlösung; und eine Entwicklungsschicht zum Entwickeln der Probenlösung, wobei
die Entwicklungsschicht Folgendes enthält: ein Markerreagenshalteteil, in dem ein Markerreagens in einem trockenen Zustand gehalten ist, sodass es durch die Entwicklung der Probenlösung eluiert werden kann; und ein Reagensfixierteil, in dem ein Ragens, das an einen Analyt binden kann und an einer Reaktion beteiligt ist, fixiert ist, sodass es nicht eluiert wird, und, wenn die Probenlösung in das Probeneinführungsteil eingeführt ist, die Probenlösung die Entwicklungsschicht zum Erreichen des Markerreagenshalteteil durchdringt und sich zu dem Reagensfixierteil bewegt, während sie das Markerreagens eluiert, wobei die Reaktion zwischen dem Analyt, dem Markerreagens und dem fixierten Reagens erfolgt, und
das Analyseverfahren umfassend: Messen einer Markerreagensmenge, die in dem Reagensfixierteil gebunden ist, wodurch der Analyt, der in der Probenlösung enthalten ist, qualitativ oder quantitativ gemessen wird, wobei
das Probeneinführungsteil mit einem raumbildenden Teil zum Ausbilden eines Raums auf der Entwicklungsschicht zum Hervorbringen eines Hohlraums versehen ist, in den die Probenlösung eingesaugt und darin gehalten wird; und
ein Zellenkontraktionswirkstoffhalteteil, das Zellenkontraktionswirkstoff hält, der Zellkomponenten zusammenzieht, in dem raumbildenden Teil enthalten ist; und
eine retikuläre Struktur, die eine einzelne Schicht mit einem Netz mit einer Porengröße von 0,1 mm bis 2 mm umfasst, in dem Probeneinführungsteil angeordnet ist, und das Blut entwickelt wird.

18. Blutbestandteilanalyseverfahren nach Anspruch 17, wobei
der Biosensor das Markerreagenshalteteil in dem Hohlraum umfasst, und
die Probenlösung in das Probeneinführungsteil eingeführt wird, die Probenlösung in der Entwicklungsschicht entwickelt wird, während das Markerreagens zum Erreichen des Reagensfixierteils eluiert wird, wodurch eine Reaktion zwischen dem Analyt, dem Markerreagens und dem fixierten Reagens erfolgt.

19. Blutbestandteilanalyseverfahren nach Anspruch 17, wobei
die retikuläre Struktur an dem Endteil des Probeneinführungsteils angeordnet wird.

20. Blutbestandteilanalyseverfahren nach Anspruch 17, wobei
die retikuläre Struktur und das Zellenkontraktionswirkstoffhalteteil derart angeordnet werden, dass die Kanten ihrer Endteile aneinander ausgerichtet gehalten sind.

21. Blutbestandteilanalyseverfahren nach Anspruch 17, wobei
ein Raum, der es der Probenlösung ermöglicht, darin zu fließen, zwischen der retikulären Struktur und dem Zellenkontraktionswirkstoffhalteteil angeordnet wird.

22. Blutbestandteilanalyseverfahren nach Anspruch 17, wobei
die retikuläre Struktur derart angeordnet wird, dass Kettfäden davon parallel zu der Richtung verlaufen, in der die Probe auf der Entwicklungsschicht entwickelt wird.

23. Blutbestandteilanalyseverfahren nach Anspruch 17, wobei
das retikuläre Gewebe aus Kunstharz hergestellt wird.

24. Blutbestandteilanalyseverfahren nach Anspruch 17, wobei
die retikuläre Struktur aus Chemiefaser, wie etwa Polyester, hergestellt wird.

25. Blutbestandteilanalyseverfahren nach Anspruch 17, wobei
die retikuläre Struktur ein Gewebe ist, das durch Weben der Chemiefaser, wie etwa Polyester, erzielt wird.

26. Blutbestandteilanalyseverfahren nach Anspruch 17, wobei
die retikuläre Struktur mit einem grenzflächenaktiven Stoff behandelt wird, sodass sie durchlässig ist.

27. Blutbestandteilanalyseverfahren nach Anspruch 17, wobei
die Probenlösung, die angewendet werden soll, Vollblut ist.

28. Blutbestandteilanalyseverfahren nach Anspruch 17, wobei
der Zellenkontraktionswirkstoff anorganisches Salz ist.

29. Blutbestandteilanalyseverfahren nach Anspruch 17, wobei
der Zellenkontraktionswirkstoff Aminosäure ist.

30. Blutbestandteilanalyseverfahren nach Anspruch 17, wobei
der Zellenkontraktionswirkstoff Saccharid ist.

31. Blutbestandteilanalyseverfahren nach Anspruch 17, wobei
der Biosensor ein immunchromatographischer Testprobekörper in einem Schritt ist.

## Revendications

1. Biocapteur réalisé en matière poreuse séchée,
ledit biocapteur comprenant : une partie d'introduction d'échantillon destinée à introduire une solution d'échantillon ; et une couche de développement destinée à développer la solution d'échantillon, où
la couche de développement comporte : une partie de maintien de réactif de marquage où un réactif de marquage est maintenu dans un état sec de façon à pouvoir être élué par le développement de la solution d'échantillon ; et une partie d'immobilisation de réactif où un réactif qui peut se lier à un analyte et qui est impliqué dans une réaction est immobilisé de sorte à ne pas être élué, et lorsque la solution d'échantillon est introduite à la partie d'introduction d'échantillon, la solution d'échantillon s'imprègne dans la couche de développement pour atteindre la partie de maintien de réactif de marquage, et se déplace vers la partie d'immobilisation de réactif tout en éluant le réactif de marquage, grâce à quoi la réaction parmi l'analyte, le réactif de marquage, et le réactif immobilisé se produit, et
une quantité de réactif de marquage lié dans la partie d'immobilisation de réactif est mesurée, mesurant ainsi qualitativement ou quantitativement l'analyte compris dans la solution d'échantillon, où
la partie d'introduction d'échantillon est pourvue d'une partie de formation d'espace pour former un espace sur la couche de développement de manière à produire une cavité dans laquelle la solution d'échantillon y est aspirée et maintenue,
une partie de maintien d'agent de contraction des cellules maintenant un agent de contraction des cellules qui contracte des composants cellulaires est comprise dans la partie de formation d'espace ; et
une structure réticulée comprenant une seule couche avec un maillage ayant une dimension des pores de 0,1 mm à 2 mm est disposée dans la partie d'introduction d'échantillon.

2. Biocapteur tel que défini dans la revendication 1, dans lequel la partie de maintien de réactif de marquage est disposée dans la cavité, et
la solution d'échantillon est introduite à la partie d'introduction d'échantillon, la solution d'échantillon est développée dans la couche de développement tandis que le réactif de marquage est élué pour atteindre la partie d'immobilisation de réactif, produisant ainsi une réaction parmi l'analyte, le réactif de marquage, et le réactif immobilisé.

3. Biocapteur tel que défini dans la revendication 1, dans lequel
la structure réticulée est disposée au niveau de la partie d'extrémité de la partie d'introduction d'échantillon.

4. Biocapteur tel que défini dans la revendication 1, dans lequel
la structure réticulée et la partie de maintien d'agent de contraction des cellules sont disposées de telle sorte que les bords de leurs parties d'extrémité soient maintenus alignés.

5. Biocapteur tel que défini dans la revendication 1, dans lequel
un espace permettant à la solution d'échantillon de s'y écouler est disposé entre la structure réticulée et la partie de maintien d'agent de contraction des cellules.

6. Biocapteur tel que défini dans la revendication 1, dans lequel
la structure réticulée est disposée de telle sorte que ses fils de chaîne soient parallèles à la direction dans laquelle l'échantillon est développé sur la couche de développement.

7. Biocapteur tel que défini dans la revendication 1, dans lequel
le tissu réticulé est réalisé en résine synthétique.

8. Biocapteur tel que défini dans la revendication 1, dans lequel
la structure réticulée est réalisée en fibre chimique telle que le polyester.

9. Biocapteur tel que défini dans la revendication 1, dans lequel
la structure réticulée est un tissu obtenu par tissage de la fibre chimique telle que le polyester.

10. Biocapteur tel que défini dans la revendication 1, dans lequel
la structure réticulée est traitée avec un agent tensio-actif de sorte à pouvoir s'imprégner.

11. Biocapteur tel que défini dans la revendication 1, dans lequel
la solution d'échantillon à appliquer est du sang.

12. Biocapteur tel que défini dans la revendication 1, dans lequel
la solution d'échantillon à appliquer est une solution comportant des bactéries.

13. Biocapteur tel que défini dans la revendication 1, dans lequel
l'agent de contraction des cellules est un sel inorganique.

14. Biocapteur tel que défini dans la revendication 1, dans lequel
l'agent de contraction des cellules est un acide aminé.

15. Biocapteur tel que défini dans la revendication 1, dans lequel
l'agent de contraction des cellules est un saccharide.

16. Biocapteur tel que défini dans la revendication 1, dans lequel
le biocapteur est un échantillon d'essai immuno-chromatographique réalisé en une seule étape.

17. Procédé permettant d'analyser un constituant sanguin en utilisant un biocapteur réalisé en matière poreuse séchée,
ledit biocapteur comprenant : une partie d'introduction d'échantillon destinée à introduire une solution d'échantillon ; et une couche de développement destinée à développer la solution d'échantillon, où
la couche de développement comporte : une partie de maintien de réactif de marquage où un réactif de marquage est maintenu dans un état sec de façon à pouvoir être élué par le développement de la solution d'échantillon ; et une partie d'immobilisation de réactif où un réactif qui peut se lier à un analyte et qui est impliqué dans une réaction est immobilisé de sorte à ne pas être élué, et lorsque la solution d'échantillon est introduite à la partie d'introduction d'échantillon, la solution d'échantillon s'imprègne dans la couche de développement pour atteindre la partie de maintien de réactif de marquage, et se déplace vers la partie d'immobilisation de réactif tout en éluant le réactif de marquage, grâce à quoi la réaction parmi l'analyte, le réactif de marquage, et le réactif immobilisé se produit, et
ledit procédé d'analyse comprenant : la mesure d'une quantité de réactif de marquage lié dans la partie d'immobilisation de réactif, mesurant ainsi qualitativement ou quantitativement l'analyte compris dans la solution d'échantillon, où
la partie d'introduction d'échantillon est pourvue d'une partie de formation d'espace pour former un espace sur la couche de développement de manière à produire une cavité dans laquelle la solution d'échantillon y est aspirée et maintenue,
une partie de maintien d'agent de contraction des cellules maintenant l'agent de contraction des cellules qui contracte des composants cellulaires est comprise dans la partie de formation d'espace ; et
une structure réticulée comprenant une seule couche avec un maillage ayant une dimension des pores de 0,1 mm à 2 mm est disposée dans la partie d'introduction d'échantillon, et le sang est développé.

18. Procédé d'analyse de constituant sanguin tel que défini dans la revendication 17, dans lequel
le biocapteur comprend la partie de maintien de réactif de marquage dans la cavité, et
la solution d'échantillon est introduite à la partie d'introduction d'échantillon, la solution d'échantillon est développée dans la couche de développement tandis que le réactif de marquage est élué pour atteindre la partie d'immobilisation de réactif, produisant ainsi une réaction parmi l'analyte, le réactif de marquage, et le réactif immobilisé.

19. Procédé d'analyse de constituant sanguin tel que défini dans la revendication 17, dans lequel
la structure réticulée est disposée au niveau de la partie d'extrémité de la partie d'introduction d'échantillon.

20. Procédé d'analyse de constituant sanguin tel que défini dans la revendication 17, dans lequel
la structure réticulée et la partie de maintien d'agent de contraction des cellules sont disposées de telle sorte que les bords de leurs parties d'extrémité soient maintenus alignés.

21. Procédé d'analyse de constituant sanguin tel que défini dans la revendication 17, dans lequel
un espace permettant à la solution d'échantillon de s'y écouler est disposé entre la structure réticulée et la partie de maintien d'agent de contraction des cellules.

22. Procédé d'analyse de constituant sanguin tel que défini dans la revendication 17, dans lequel
la structure réticulée est disposée de telle sorte que ses fils de chaîne soient parallèles à la direction dans laquelle l'échantillon est développé sur la couche de développement.

23. Procédé d'analyse de constituant sanguin tel que défini dans revendication 17, dans lequel
le tissu réticulé est réalisé en résine synthétique.

24. Procédé d'analyse de constituant sanguin tel que défini dans la revendication 17, dans lequel
la structure réticulée est réalisée en fibre chimique telle que le polyester.

25. Procédé d'analyse de constituant sanguin tel que défini dans la revendication 17, dans lequel
la structure réticulée est un tissu obtenu par tissage de la fibre chimique telle que le polyester.

26. Procédé d'analyse de constituant sanguin tel que défini dans la revendication 17, dans lequel
la structure réticulée est traitée avec un agent tensioactif de sorte à pouvoir s'imprégner.

27. Procédé d'analyse de constituant sanguin tel que défini dans la revendication 17, dans lequel
la solution d'échantillon à appliquer est du sang total.

28. Procédé d'analyse de constituant sanguin tel que défini dans la revendication 17, dans lequel
l'agent de contraction des cellules est un sel inorganique.

29. Procédé d'analyse de constituant sanguin tel que défini dans la revendication 17, dans lequel
l'agent de contraction des cellules est un acide aminé.

30. Procédé d'analyse de constituant sanguin tel que défini dans la revendication 17, dans lequel
l'agent de contraction des cellules est un saccharide.

31. Procédé d'analyse de constituant sanguin tel que défini dans la revendication 17, dans lequel
le biocapteur est un échantillon d'essai immuno-chromatographique réalisé en une seule étape.
